(19) Europäisches Patentamt
European Patent Office
Office européen des brevets

(11) EP 2 827 865 B1

(12) EUROPEAN PATENT SPECIFICATION

(45) Date of publication and mention of the grant of the patent:
**12.12.2018 Bulletin 2018/50**

(21) Application number: **13721012.6**

(22) Date of filing: **20.03.2013**

(51) Int Cl.:
*A61K 31/4425* (2006.01) *A61K 31/5375* (2006.01)
*A61P 31/04* (2006.01) *A61P 31/10* (2006.01)
*A61K 8/02* (2006.01) *A61K 8/04* (2006.01)
*A61K 8/49* (2006.01) *A61Q 11/00* (2006.01)
*A61Q 17/00* (2006.01)

(86) International application number:
**PCT/GB2013/050728**

(87) International publication number:
**WO 2013/140170 (26.09.2013 Gazette 2013/39)**

(54) **TREATMENT OF MICROBIAL INFECTIONS**

BEHANDLUNG VON MIKROBIELLEN INFEKTIONEN

TRAITEMENT D'INFECTIONS MICROBIENNES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.03.2012 GB 201204864**
**21.03.2012 GB 201204970**

(43) Date of publication of application:
**28.01.2015 Bulletin 2015/05**

(73) Proprietor: **Merial, Inc.**
**Duluth, GA 30096 (US)**

(72) Inventor: **YOULTON, Simon**
**Chester CH4 9QZ (GB)**

(74) Representative: **D Young & Co LLP**
**120 Holborn**
**London EC1N 2DY (GB)**

(56) References cited:
**WO-A1-92/08442** **WO-A1-2006/082393**
**WO-A1-2007/060413** **WO-A2-2009/013475**

- **BERESWILL S ET AL: "Susceptibility in vitro of Helicobacter pylori to cetylpyridinium chloride.", FEMS IMMUNOLOGY AND MEDICAL MICROBIOLOGY JUN 1999, vol. 24, no. 2, June 1999 (1999-06), pages 189-192, XP002697943, ISSN: 0928-8244**
- **"Martindale, The complete drug reference", 2000, Pharmaceutical Press, XP002697944, page 1105 "Cetrimide" to page 1106 "Cetylpyridinium chloride"**
- **ELWORTHY A J ET AL: "ANTIMICROBIAL PROPERTIES OF DELMOPINOL AGAINST ORAL BACTERIA", LETTERS IN APPLIED MICROBIOLOGY, OXFORD, GB, vol. 20, no. 3, 1 January 1995 (1995-01-01), pages 191-194, XP008066666, ISSN: 1472-765X**

EP 2 827 865 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the Invention

The present invention relates to compositions useful in the prevention and/or treatment of microbial infection.

### Background

The living body may be infected by a wide variety of microbes. The mucosa and the surface of the skin are particularly at risk of infection given their exposure to the external environment. When the natural barrier provided by the mucosa and the skin is broken by injury or surgery, microbes can spread throughout the layers of the skin and mucosa, which can lead to infection of the wound site. This is particularly the case following invasive surgery. In this instance, the surgical wound may become infected by microbes naturally present in the environment, on the surface of the skin or mucosa, or on the surgical instruments used in the procedure. The infection of wounds causes the healing process to be extended and, in some cases, where wounds are infected, they may fail to heal.

Morpholino compounds are known in the art to have low antibacterial activity. These compounds alone are particularly effective at inhibiting the formation of biofilms, and have been used in this context in WO 06/082393 in compositions to be applied to abiotic surfaces. Synergistic combinations of morpholino compounds with other agents have also been used, for example in the context of dental care, where the inclusion of chelating agents has been found to be effective against the growth of oral plaque bacteria (US 5,147,632). WO 2007/060413 describes the use of morpholino compounds in the treatment of an infected sub-gingival pocket. WO 2009/013475 describes the use of morpholino compounds for the promotion of healing of a wound.

The antimicrobial activity of quaternary ammonium compounds, such as cetylpyridinium salts, is also known in the art. JP 2002256155 discloses the antimicrobial activity of cetylpyridinium salts alone against oral plaque bacteria, whereas JP 8151325 and US 2003/0211053 disclose synergistic antimicrobial combinations of the salts with lysine derivatives and proteolytic enzymes respectively, with the latter formulated for the treatment of plaque and halitosis. Bereswill et al (1999) FEMS Immunology and Medical Microbiology 24: 189-92, describes the susceptibility of *Helicobacter pylori* to cetylpyridinium chloride. Furthermore, WO 92/08442 describes compositions comprising morpholino compounds in combination with a range of antimicrobial agents (including cetylpyridinium salts), for use in treating or preventing plaque and gingivitis in the oral cavity; wherein such compositions are demonstrated as having inhibitory activity on the growth of two bacterial species associated with dental plaque (*Streptococcus mutans* and *Bacteriodes melaninogenicus*).

Accordingly, there is an overwhelming bias in the prior art towards the use of cetylpyridinium salts and morpholino compounds in the treatment of mild disorders of the oral cavity, such as halitosis and plaque. The nature of microbial colonisation in different pathologies is dependent on a number of factors, including the associated microbes, and environmental factors such as temperature, nutrient and fluid levels, and pH. Dental plaque comprises largely gram-positive cocci bacteria in the form of a polymicrobial biofilm on the tooth surface. With regard to environmental factors, in species such as S. *mutans,* the presence of sucrose in the oral cavity has been found to enhance initial bacterial adherence and subsequent colonisation.

The nature of microbial infection in wounds and periodontitis is distinct from that of plaque, by virtue of the differences in associated microbial species, physiological conditions, and severity of the conditions. James et al. (Wound Rep Reg. 2008. 16. 37-44) have argued the former two conditions as being analogous in their pathological mechanism, due to the role of diverse microbial communities acting in consort over time to cause a chronic infection. Severe adult periodontitis is largely characterised by the presence of the gram-negative anaerobe *Porphyromonas gingivalis* in periodontal lesions, whereas infected wounds may be colonised by a variety of microbes, including bacteria from the *Staphylococcus* and *Enterococcus* genera. There is consequently a need to provide compositions which are effective in the treatment of the above chronic infections, which must have efficacious antimicrobial properties against the species implicated in these disorders.

### Summary of the Invention

The present invention is based on the finding that compositions comprising compounds of formula (I) and a cetylpyridinium salt possess synergistically improved abilities to treat and/or prevent infections caused by a range of microbial species, or that compounds of formula (I) act to potentiate the antimicrobial effects of the cetylpyridinium salt against said species. These microbial species are known in the art to be associated with disorders such as chronic infections of skin or mucosal wounds and periodontal lesions. Therefore, in contrast to the above-mentioned prior art, which shows efficacy against bacteria associated with plaque or gingivitis, the present invention provides for the treatment or prevention of infections in wounds of the skin or mucosa.

According to a first aspect of the invention, the present invention provides a composition comprising a morpholino compound in combination with a cetylpyridinium salt, the morpholino compound having the general formula (I)

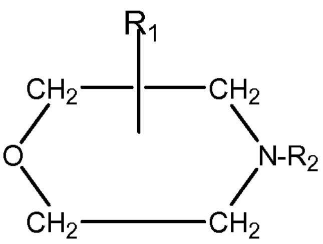

(I)

wherein R1 is a straight or branched alkyl group containing 8 to 16 carbon atoms at the 2- or 3- position of the morpholino ring, and R2 is a straight or branched alkyl group containing 2 to 10 carbon atoms, substituted with a hydroxyl group except in the alpha-position, or pharmaceutically acceptable salts thereof for use in the treatment or prevention of an infection in a patient caused by *Staphylococcus spp,* , wherein the infection is in a wound of the skin or mucosa of a patient.

According to a second aspect of the invention, the present invention provides a composition for use as defined above for use in treating or preventing a post-operative infection in or on the skin or mucosa of a patient.

According to a third aspect of the invention, the present invention provides a composition for use as defined above for use in pre-operatively reducing the amount of infection-causing microbes present in or on the skin or mucosa of a patient.

## Description of the Figures

The invention is described with reference to the accompanying figure, wherein:
Figure 1 shows Fractional inhibitory concentration (FIC) index value ranges used to classify whether a compound potentiates or synergistically improves the antimicrobial effect of a given compound.

## Detailed Description of the Invention

The present invention provides novel uses of compositions comprising a compound of formula (I) and a cetylpyridinium salt. The above components have been found to display synergistic antimicrobial activity against microbes which cause infection or conditions in or on the skin or mucosa of a patient, thus indicating their suitability for use in treating such disorders.

The morpholino alcohols can be prepared by several processes as described in US Patent No. 4636382 such as:

(a) by alkylating a morpholino derivative having the formula

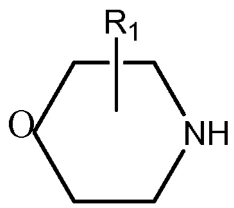

wherein $R_1$ is as defined above; with an alkylating agent of the formula

$R_2X$

wherein $R_2$ is as defined above and X is halogen or an organic sulfonic ester, or wherein X together with a hydroxyl group present in $R_2$ is a reactive oxide;

(b) by ring closure of a compound having the general formula

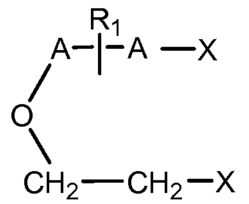

wherein $R_1$ is as defined above, X is halogen or an organic sulfonic ester and A represents $CH_2$ groups, one $CH_2$ group being substituted with the group $R_1$; with an amino alkanol of the general formula

$NH_2R_2$

wherein $R_2$ is as defined above;

(c) by reducing a mono- or di-oxo substituted morpholine having the general formula

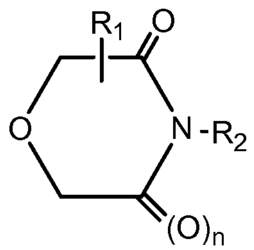

wherein $R_2$ is as defined above, n is 0 or 1, and $R_1$ is as defined above and is at the 2-position when n is 1 and at the 2- or 3-position when n is 0; or

(d) by starting from a morpholino compound having the general formula

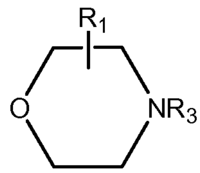

wherein $R_1$ is as defined above and $R_3$ is a straight or branched alkyl group containing a group transformable to OH or $CH_2OH$; especially

(d1) by converting a compound of the formula VIII, wherein the group in $R_3$ transformable to OH is halogen, NHAc, OAc, O-alkyl, $O-CH_2C_6H_5$; or
(d2) by converting a compound of the formula VIII, wherein the group in $R_3$ transformable to $CH_2OH$ is $-COOC_2H_5$, -CN, -CHO; or
(d3) $R_3$ represents $-CO(CH_2)_n-COOC_2H_5$ (n=0-8).

The morpholino compound may also be prepared by the method as described in WO 2007/057681 and WO 2007/091009.

The morpholino compound used in this invention can be used in its free base form or as a pharmaceutically-acceptable salt. Examples of pharmaceutically-acceptable salts are the salts of acids such as acetic acid, phosphoric acid, boric acid, hydrochloric acid, maleic acid, benzoic acid, citric acid, maleic acid, oxalic acid, tartaric acid, succinic acid, glutaric acid, gentisic acid, valeric acid, gallic acid, beta-resorcyclic acid, acetyl salicylic acid, salicylic acid, perchloric acid, barbituric acid, sulfanilic acid, phytic acid, p-nitro benzoic acid, stearic acid, palmitic acid, oleic acid, myristic acid, lauric acid; however others known to the skilled person may be used. The most preferred salts are those of hydrochloric acid.

The most preferred morpholino compound used in the present invention is delmopinol, which has the non-proprietory name (INN) of (3-(4-propylheptyl)-4-(2-hydroxyethyl)morpholine) with CAS No. 79874. Equally most preferred

is the hydrochloride salt of delmopinol.

The claimed morpholino compounds are known *per se,* as described in US 4894221 and US 5082653.

The antimicrobial agent used in the compositions of the present invention is a cetylpyridinium salt. The present inventors have found that the combination of a compound of formula (I) with a cetylpyridinium salt acts synergistically to treat or prevent microbial infection in or on the skin or mucosa, or related conditions in a patient. The cetylpyridinium salt may be used in the form of a pharmaceutically-acceptable salts of acids such as acetic acid, phosphoric acid, boric acid, citric acid, malic acid, oxalic acid, tartaric acid, succinic acid, gluratic acid, gentisic acid, valeric acid, gallic acid, beta-resorcyclic acid, acetylsalicyclic acid, salicyclic acid, perchloric acid, barbituric acid, sulfonic acid, phytic acid p-nitro benzoic acid, stearic acid, palmitic acid, oleic acid, myristic acid, lauric acid and others known to the skilled person. Preferably the cetylpyridinium salt is a halide salt and more preferably, the cetylpyridinium salt is cetylpyridinium chloride.

In the composition of the present invention, the morpholino compound is present preferably in an amount ranging from about 0.005% to about 20.0% by total weight of the composition, preferably from about 0.005% to 5%, more preferably from about 0.01% to about 1.0%, and most preferably from about 0.05% to about 0.2%. The amount of cetylpyridinium salt in the composition preferably ranges from about 0.001% to about 5.0% by total weight of the composition, more preferably from about 0.002% to about 2.0%; and most preferably from about 0.005% to about 1.0%. In one embodiment, the morpholino compound and cetylpyridinium salt are provided in a 1:1 weight ratio. Preferably, in said embodiment, the morpholino compound is provided at 0.1% and the cetypyridinium salt is provided at 0.1% of the total weight of the composition.

Infections or conditions caused by microbes can be reduced or prevented using the compositions of the present invention; wherein the antimicrobial action of the components of said compositions is synergistic compared to that of known compositions comprising a compound of formula (I) and other antimicrobial agents. Accordingly, compounds of formula (I) act to potentiate the antimicrobial effect of the cetylpyridinium salt. The term "microbe" includes bacteria, fungi and yeast. Microbial species against which compositions of the present invention may be effective may be gram-positive, gram-negative, aerobic or anaerobic. The microbial species against which compositions of the present invention are effective include *Staphylococcus spp.* In particular, the compositions of the invention are for use in treating or preventing an infection caused by *Staphylococcus spp.,* such as S. *aureus.*

As used herein, the nomenclature "spp", following a given microbial genus, refers to any microbial species belonging to said genus, as is common practice in the art.

In the context of the present invention, an infection or condition in a patient may be considered as being "caused" by a given microbe or combination of microbes, when the absence of said microbe or combination would be understood by the skilled person to ameliorate the infection or condition, when compared with the presence of said microbe or combination. Accordingly, the skilled person would understand the microbe or combination of microbes to exert a pathological effect in the context of the infection or condition. Preferably, said microbe or combination would be understood by the skilled person to provide greater amelioration of the infection or condition if removed, than all other known pathological microbes (in the context of the infection or condition) if removed. More preferably, said microbe or combination would be understood by the skilled person to provide greater amelioration of the infection or condition if removed, than all other known pathological agents (in the context of the infection or condition) if removed. Said microbe or combination does not have to be the sole pathological agent in the infection or condition, although this is most preferable.

The microbes against which the compositions of the present invention are particularly effective are those which infect the skin or mucosa such as the lining of the oral cavity, nasal passages, lips, eyelids, ears, genitalia, and anus, or those which may be found on the surface of the skin. In particular, the components of compositions of the present invention are synergistically effective against microbes which cause infections in wounds in the skin and or mucosa. Preferably, compositions of the present invention are effective at treating or preventing infections in wounds of the non-oral mucosa. The compositions of the present invention may therefore be used to treat or prevent infections in such wounds.

The microbes against which the compositions of the present invention are effective may be involved in the pathogenesis of periodontitis.

In the context of the present invention, a wound is understood to mean any breach of the surface of the skin or mucosa which may be caused by physical injury, such as caused when the skin or mucosa is physically penetrated or damaged by a burn or caused by infection or disease. The wound may be superficial or may be deeper, penetrating several layers of the mucosa or skin. Preferably the wound is in the skin.

The infection of the wound in the skin or mucosa may include the microbial genera *Staphylococcus spp, Enterococcus spp, Prevotella spp, Cornybacterium spp, Actinomyces spp, Pseudomonas spp, Porphyromonas spp, Finegoldia spp, Peptostreptococcus spp, Anaerococcus spp, Serratia spp* or combinations thereof; particularly the species *S. aureus, E. faecalis, E. coli, C. albicans* or combinations thereof.

Periodontitis is a condition in which the gums are pulled away from the teeth to form pockets that are open to infection (periodontal lesions). The condition can lead to bone loss from around the tooth root and jaw. Microbes which are implicated in causing periodontitis include the genera *Prevotella. spp, Actinomyces spp, Porphyromonas spp, Candida*

*spp* or combinations thereof, particularly the species *P. gingivalis* and *C. albicans* or combinations thereof.

The compositions of the present invention may be used pre-operatively to treat the skin or mucosa at the site at which an incision is to be made. Preferably, the area of the body that is contacted with the composition of the present invention comprises the site of contact between the body and the surgical instrument (or a portion of said site), and/or the immediate surrounding area.

The healing of wounds caused by injury or surgical incision may be limited if the wound is infected by a microbe. Compositions of the present invention may help promote wound healing by preventing or treating the microbial infection at the site of the wound. Preferably, the wound is treated with the composition of the present invention post-injury or post-operatively. Preferably the wound is in the oral cavity or the skin. Compositions of the present invention may be used pre-operatively to sterilise the surface of the body in which an incision is to be made and/or may be used post-operatively to prevent infection or to treat an infection at the incision site.

More preferably, the infection is caused by contacting the body with a medical device. The compositions of the present invention may be used to treat an abiotic surface, such as that of a medical device, before, during or after, preferably immediately after, initial contact between a medical device and the body. A medical device may include a surgical instrument.

In a preferred embodiment, a composition of the present invention is used to prevent or reduce infection caused by planktonic microbes. As used herein, the term planktonic refers to microbes that are not attached to a surface; planktonic microbes are free floating. These microbes are not part of a biofilm, but may be the same species as microbes comprising a biofilm. A composition of the present invention can be used to act on such microbes before they attach to the human body.

The compound of formula (I) and the cetylpyridinium salt comprising the composition of the invention may be applied to a given site simultaneously, such that the composition is formed prior to administration, or sequentially (in any order), such that the composition is formed upon application of the second (later applied) component.

The composition of the invention may be in the form of a medicament comprising pharmaceutically acceptable excipients that are well known in the art. The composition of the invention may be formulated to additionally comprise sweeteners, flavourants and/or colorants particularly when the composition is intended for oral use. Natural sweeteners, flavourants and/or colorants are known in the art.

The composition of the invention may be delivered to the skin or mucosa in any suitable form or amount that achieves the desired effect. Preferably, the composition is formulated as a gel, foam, cream, emollient, spray, wipe mouthwash, toothpaste, chew or gum. The compositions of the present invention may be applied to the oral mucosa by various methods including, but not limited to brushing, spraying, painting or rinsing of the oral cavity with the composition. The compositions of the present invention may be applied to the skin by topical application, for example through the use of a dressing comprising the composition, which contacts the skin; preferably wherein the dressing is suitable for contacting a wound, for example wherein the dressing is a bandage, gauze, plaster, hydrogel or other medical dressing. Alternatively, the composition may be applied to the skin as an aqueous wash, for example by way of a wound irrigation wash comprising the composition. The composition may also be applied to the skin as a gel or spray.

The following Examples illustrate the invention. The invention is defined by the claims. Any subject-matter falling outside the scope of the claims is provided for information purposes only.

## Examples

The effect on the antimicrobial ability of compositions comprising delmopinol with or without cetylpyridinium chloride (CPC) was studied. Manufacturing details of CPC are provided below:

| Compound | CAS | Supplier | Cat. No. | Purity | Solvent |
|---|---|---|---|---|---|
| CPC | 123-03-5 | MP Biomedicals | 0219017725 | 99.8% | $dH_2O$ |

The compositions of the invention were tested as a solution having the following base composition to which delmopinol and/or CPC was added:
Broth A was prepared, which comprises a base formulation intended for use in oral care applications but without delmopinol or CPC (below) were produced separately then mixed and stirred thoroughly before the final pH was adjusted to 5.6 to 5.7 using 1 M NaOH/HCl as required. The final mixture was then sterilized by filtration using a 0.45 $\mu$M PES filter (Nalgene, 169-0045).

Bacterial preparations were prepared using freshly grown cultures on solid media. Several loop-fulls of culture were transferred to 10-20 ml of the appropriate broth and vortexed gently until a fine suspension was produced. The initial $OD_{600}$nm of the cell suspensions was measured and adjusted to 0.2 to give a colony count of $1\times10^8$ cfu/ml. Final

inocula were prepared by diluting the adjusted cell suspensions in appropriate broth as detailed below:

| Microbe | Dilution |
|---|---|
| *C. albicans* NCTC 1363 | 1 in 1000 |
| *Ent. faecalis* ATCC 29212 | 1 in 100 |
| *E. coli* ATCC 25922 | 1 in 100 |
| *P. gingivalis* NCTC 11834 | 1 in 20 |
| *Prv. nigrescens* ATCC 33563 | 1 in 20 |
| S. *aureus* ATCC 29213 | 1 in 200 |

**Antimicrobial Activity of delmopinol or CPC alone**

The antimicrobial activity of CPC and delmopinol was determined in both standard media and Broth A. 195 μl of normal broth (standard assay) or broth A described above was added to a 96 well plate. The broths were added to wells in columns 1, 11 (negative control, broth only) and 12 (positive control).

100 μl of normal broth (standard assay) or broth A was added to wells in columns 2-10. 5 μl of either a CPC or delmopinol stock (prepared in a suitable solvent to 80x the desired final concentration) was then added to wells in column 1 and mixed thoroughly (minimum three replicates). Doubling dilutions were prepared by transferring 100 μl of the mixture from column 1 into column 2, then column 2 to column 3 and so on until column 10.

After mixing in column 10, 100 μl was discarded. 5 μl of the appropriate solvent was added to wells in column 12, mixed and 100 μl discarded.

Finally, 100 μl of inoculum (prepared in either normal broth or 2x broth A) was added to the wells in columns 1-10 and 12 and the plates were incubated as described above.

Following the incubation period, plates were examined for growth by eye. The first well of each row that contained no growth was marked at the minimum inhibitory concentration (MIC).

5 μl samples from all wells exhibiting no growth and the first well exhibiting growth were transferred to an appropriate agar plate, allowed to soak into the agar and then incubated. Following this incubation period plates were examined and the lowest concentration of test compound where no growth was recorded as the minimum bactericidal concentration (MBC).

**Antimicrobial activity of delmopinol and CPC in combination**

Assays were set up as described above except that:
A delmopinol stock solution (in $dH_2O$) prepared to 200x the desired final concentration (this would be ¼ of its recorded MIC alone) was diluted 1 in 100 into the inocula and then 100 μl immediately added to all wells in columns 1-10 and 12.

Rows A - C record MIC/MBC for the test compound alone and rows F - H record MIC/MBC of test the compound in combination with a ¼ MIC of delmopinol.

The mode of interaction between CPC and delmopinol was interpreted by converting MIC data into fractional inhibitory concentration index (FICi) score using the following formula:

$$FIC_{(CPC)} = \text{MIC of CPC with delmopinol/MIC of CPC alone}$$

$$FIC_{(Delmopinol)} \text{ is fixed at 0.25 (i.e. ¼ MIC)}$$

$$FICi = FIC_{(CPC)} + FIC_{(Delmopinol)}$$

The FICi was then interpreted using the following parameters (this is depicted in Figure 1):

Synergy = FICi ≤0.5
Potentiation = FICi >0.5 - 0.75
Indifferent = FICi >0.75 - 4

Antagonism = FICi >4

A fractional bactericidal concentration index (FBCi) was calculated and interpreted in the same manner but using MBC data. The $FBC_{(Delmopinol)}$, although fixed, would be less than or equal to 0.25 depending on what fraction of ¼ MIC concentration is relative to its MBC alone.

**Antimicrobial activity of CPC and delmopinol combinations (Chequerboard assay)**

Separate CPC and delmopinol stocks were prepared in $dH_2O$ to 80x the desired final concentration and then a series of two fold dilutions were prepared in $dH_2O$.

190 μl of normal broth (standard assays) or broth A was added to wells of a 96 well plate. 5 μl of each dilution of CPC was added to the appropriate wells of the 96 well plate. 5 μl of each dilution of delmopinol was added to the appropriate wells of the 96 wells plate. The contents of each well were mixed thoroughly and then 100 μl volume was discarded.

100 μl of inocula was added to all wells in columns 1 - 10 and 12. Plates were then incubated as described above. Following the incubation period, plates were examined for growth by eye and all wells were recorded for the presence or absence of growth (used for FIC determination). 5 μl samples of all wells were transferred to an appropriate agar plate, allowed to soak in agar and then incubated. Following incubation the plates were examined and growth/no growth recorded for every well sample (used for FBC determination).

$$FIC_{(CPC)} = \text{MIC of CPC with delmopinol/MIC of CPC alone}$$

$$FIC_{(Delmopinol)} = \text{MIC of delmopinol with CPC/MIC of delmopinol alone}$$

$$FICi = FIC_{(CPC)} + FIC_{(Delmopinol)}$$

FBCi were calculated in the same manner but using MBC data.

The FICi and FBCi were interpreted using the same parameters as set out above.

**Antimicrobial activity of Decapinol and CPC in combination**

Delmopinol is commercially available as a mouthwash and is sold under the trademark Decapinol. Decapinol (for testing delmopinol plus CPC) was reformulated to contain 1:1 ratio of CPC to delmopinol i.e. 0.1% each. Decapinol without delmopinol (for testing CPC alone) was also reformulated.

Further formulations of complete Decapinol with CPC and Decapinol without delmopinol but with CPC were made to give a ratio of DEL:CPC of 50:1.

195 μl of Broth A was added to wells in column 11 (negative control) of a 96 well round bottom plate. 100 μl of Broth A was added to wells in column 2-10 and 12 (positive control).

200 μl of the adjusted ratio formulations (Decapinol +/- delmopinol), 1:1 ratio formulations (Decapinol +/- delmopinol and diluted 1 in 16 in Broth A) or complete Decapinol (for testing delmopinol alone) were added to wells in column 1.

Doubling dilutions were prepared by transferring 100 μl of the mixture from column 1 into column 2, then column 2 to column 3 and so on until column 10. 100 μl of inocula (prepared in 2x broth) was added to all wells in columns 1 - 10 and 12. Plates were then incubated as described in Table 1.

Following the incubation period, plates were examined for growth by eye. The first well of each row that contained new growth was marked as the minimum. The first well of each row that contained no growth was marked as the MIC.

5 μl samples from all wells exhibiting no growth and the first well exhibiting growth were transferred to an appropriate agar plate, allowed to soak in agar and then incubated. Plates were then examined and the lowest concentration of test compound where no growth occurred was recorded as the MBC.

The mode of interaction between CPC and delmopinol was defined by converting MIC and MBC data into FICi and FBCi and interpreted using the parameters described above.

**Results and Discussion**

Tables 1-12 show the antimicrobial mode of action (potentiation or synergy) of delmopinol and cetylpyridinium chloride (CPC) on *Porphyromonas gingivalis* (Tables 1 and 2), *Prevotella nigrescence* (Tables 3 and 4), *Staphylococcus aureus* (Tables 5 and 6), *Entercoccus faecalis* (Tables 7 and 8), *Escherichia coli* (Tables 9 and 10), *Candida albicans* (Tables 11 and 12). The results are summarised in Table 13.

**Table 1**

| *P. gingivalis* | | | | | | | | | | | | |
|---|---|---|---|---|---|---|---|---|---|---|---|---|
| | Activity alone (ug/ml) | | | | Combination with delmopinol (FIC) | | | | Evaluation in Decapinol | | | |
| Compound | Standard | | Decapinol* base | | Standard | | Decapinol* base | | 1:1 Ratio | | Adjusted ratio^ | |
| | MIC | MBC | MIC | MBC | FICi | FBCi | FICi | FBCi | FICi | FBCi | FICi | FBCi |
| CPC | 0.49 | 0.49 | 0.49 | 0.49 | 0.62p | 0.75p | 1.5 | NEP | 1.01 | 1.01 | 0.75p | 0.75p |
| Delmopinol | 1.95 | 31.25 | 31.25 | 31.25 | | | | | | | | |

* No delmopinol or sodium benzoate present
^ Ratios: 50:1 (Del:CPC)
p = potentiation

**Table 2**

| *P. gingivalis* | | | | | | | | | | | | |
|---|---|---|---|---|---|---|---|---|---|---|---|---|
| | Activity alone (uM) | | | | Combination with delmopinol (FIC) | | | | Evaluation in Decapinol | | | |
| Compound | Standard | | Decapinol* base | | Standard | | Decapinol* base | | 1:1 Ratio | | Adjusted ratio^ | |
| | MIC | MBC | MIC | MBC | FICi | FBCi | FICi | FBCi | FICi | FBCi | FICi | FBCi |
| CPC | 1.4 | 1.4 | 1.4 | 1.4 | 0.62p | 0.75 | 1.5 | NEP | 1.01 | 1.01 | 0.75p | 0.75p |
| Delmopinol | 10 | 120 | 120 | 120 | | | | | | | | |

* No delmopinol or sodium benzoate present
^ Ratios: 50:1 (Del:CPC)
p = potentiation

**Table 3**

| *Prv. nigrescence* | | | | | | | | | | | | |
|---|---|---|---|---|---|---|---|---|---|---|---|---|
| | Activity alone (ug/ml) | | | | Combination with delmopinol (FIC) | | | | Evaluation in Decapinol | | | |
| Compound | Standard | | Decapinol* base | | Standard | | Decapinol* base | | 1:1 Ratio | | Adjusted ratio^ | |
| | MIC | MBC | MIC | MBC | FICi | FBCi | FICi | FBCi | FICi | FBCi | FICi | FBCi |
| CPC | 0.06 | 0.49 | 0.25 | 0.49 | 0.75p | 0.79 | 0.5s | 0.53p | 1 | 1 | 0.38s | 1.5 |
| Delmopinol | 62.5 | 62.5 | 31.25 | 125 | | | | | | | | |

* No delmopinol or sodium benzoate present
^ Ratios: 50:1 (Del:CPC)
p = potentiation
s = synergy

**Table 4**

| *Prv. nigrescence* | | | | | | | | | | | | |
|---|---|---|---|---|---|---|---|---|---|---|---|---|
| | Activity alone (uM) | | | | Combination with delmopinol (FIC) | | | | Evaluation in Decapinol | | | |
| Compound | Standard | | Decapinol* base | | Standard | | Decapinol* base | | 1:1 Ratio | | Adjusted ratio^ | |
| | MIC | MBC | MIC | MBC | FICi | FBCi | FICi | FBCi | FICi | FBCi | FICi | FBCi |
| CPC | 0.2 | 1.4 | 0.7 | 1.4 | 0.75p | 0.79 | 0.5s | 0.53p | 1 | 1 | 0.38s | 1.5 |
| Delmopinol | 230 | 230 | 120 | 460 | | | | | | | | |

* No delmopinol or sodium benzoate present
^ Ratios: 50:1 (Del:CPC)
p = potentiation
s = synergy

**Table 5**

| S. *aureus* | | | | | | | | | | | | |
|---|---|---|---|---|---|---|---|---|---|---|---|---|
| | Activity alone (ug/ml) | | | | Combination with delmopinol (FIC) # | | | | Evaluation in Decapinol | | | |
| Compound | Standard | | Decapinol* base | | Standard | | Decapinol* base | | 1:1 Ratio | | Adjusted ratio^ | |
| | MIC | MBC | MIC | MBC | FICi | FBCi | FICi | FBCi | FICi | FBCi | FICi | FBCi |
| CPC | 3.9 | 7.8 | 0.49 | 1.95 | 0.38s | 0.63p | 0.38s | 0.75p | 1 | 0.5s | 0.63P | 0.38s |
| Delmopinol | 125 | 250 | 250 | 250 | | | | | | | | |

* No delmopinol or sodium benzoate present
^ Ratios: 50:1 (Del:CPC)
p = potentiation
s = synergy
# 1/4 delmopinol expts (Not full FIC/FBC)

**Table 6**

| *S. aureus* | | | | | | | | | | | | |
|---|---|---|---|---|---|---|---|---|---|---|---|---|
| | Activity alone (uM) | | | | Combination with delmopinol (FIC) # | | | | Evaluation in Decapinol | | | |
| Compound | Standard | | Decapinol* base | | Standard | | Decapinol* base | | 1:1 Ratio | | Adjusted ratio^ | |
| | MIC | MBC | MIC | MBC | FICi | FBCi | FICi | FBCi | FICi | FBCi | FICi | FBCi |
| CPC | 11.5 | 22.9 | 1.4 | 5.7 | 0.38s | 0.63p | 0.38s | 0.75p | 1 | 0.5p | 0.63p | 0.38s |
| Delmopinol | 460 | 920 | 920 | 920 | | | | | | | | |

* No delmopinol or sodium benzoate present
^ Ratios: 50:1 (Del:CPC)
p = potentiation
s = synergy
# 1/4 delmopinol expts (Not full FIC/FBC)

**Table 7**

| *E. faecalis* | | | | | | | | | | | | |
|---|---|---|---|---|---|---|---|---|---|---|---|---|
| | Activity alone (ug/ml) | | | | Combination with delmopinol (FIC)# | | | | Evaluation in Decapinol | | | |
| Compound | Standard | | Decapinol* base | | Standard | | Decapinol* base | | 1:1 Ratio | | Adjusted ratio^ | |
| | MIC | MBC | MIC | MBC | FICi | FBCi | FICi | FBCi | FICi | FBCi | FICi | FBCi |
| CPC | 1.95 | 15.6 | 0.98 | 3.9 | ND | ND | 1.25 | 1.25 | 1 | 1.02 | 0.75p | 1.5 |
| | | | | | | | | | | | | |
| Delmopinol | 250 | 250 | 250 | 250 | | | | | | | | |

* No delmopinol or sodium benzoate present
^ Ratios: 50:1 (Del:CPC)
p = potentiation
s = synergy
# 1/4 delmopinol expts (Not full FIC/FBC)

**Table 8**

| *E. faecalis* | | | | | | | | | | | | |
|---|---|---|---|---|---|---|---|---|---|---|---|---|
| | Activity alone (uM) | | | | Combination with delmopinol (FIC)# | | | | Evaluation in Decapinol | | | |
| Compound | Standard | | Decapinol* base | | Standard | | Decapinol* base | | 1:1 Ratio | | Adjusted ratio^ | |
| | MIC | MBC | MIC | MBC | FICi | FBCi | FICi | FBCi | FICi | FBCi | FICi | FBCi |
| CPC | 5.7 | 45.9 | 2.9 | 11.5 | ND | ND | 1.25 | 1.25 | 1 | 1.02 | 0.75p | 1.5 |
| | | | | | | | | | | | | |
| Delmopinol | 920 | 920 | 920 | 920 | | | | | | | | |

* No delmopinol or sodium benzoate present
^ Ratios: 50:1 (Del:CPC)
p = potentiation
s = synergy
# 1/4 delmopinol expts (Not full FIC/FBC)

**Table 9**

| *E. coli* | Activity alone (ug/ml) | | | | Combination with delmopinol (FIC)# | | | | Evaluation in Decapinol | | | |
|---|---|---|---|---|---|---|---|---|---|---|---|---|
| Compound | Standard | | Decapinol* base | | Standard | | Decapinol* base | | 1:1 Ratio | | Adjusted ratio^ | |
| | MIC | MBC | MIC | MBC | FICi | FBCi | FICi | FBCi | FICi | FBCi | FICi | FBCi |
| CPC | 15.6 | 31.25 | 15.6 | 62.5 | 0.75p | 1.25 | 0.75p | 1.25 | 1.06 | 1.06 | 1.25 | 1.25 |
| | | | | | | | | | | | | |
| Delmopinol | 250 | 250 | 250 | 250 | | | | | | | | |

* No delmopinol or sodium benzoate present
^ Ratios: 50:1 (Del:CPC)
p = potentiation
s = synergy
# 1/4 delmopinol expts (Not full FIC/FBC)

**Table 10**

| *E. coli* | | | | | | | | | | | | |
|---|---|---|---|---|---|---|---|---|---|---|---|---|
| | Activity alone (uM) | | | | Combination with delmopinol (FIC)# | | | | Evaluation in Decapinol | | | |
| Compound | Standard | | Decapinol* base | | Standard | | Decapinol* base | | 1:1 Ratio | | Adjusted ratio^ | |
| | MIC | MBC | MIC | MBC | FICi | FBCi | FICi | FBCi | FICi | FBCi | FICi | FBCi |
| CPC | 45.9 | 91.9 | 45.9 | 183.8 | 0.75p | 1.25 | 0.75p | 1.25 | 1.06 | 1.06 | 1.25 | 1.25 |
| | | | | | | | | | | | | |
| Delmopinol | 920 | 920 | 920 | 920 | | | | | | | | |

* No delmopinol or sodium benzoate present
^ Ratios: 50:1 (Del:CPC)
p = potentiation
s = synergy
# 1/4 delmopinol expts (Not full FIC/FBC)

**Table 11**

| *C. albicans* | | | | | | | | | | | | |
|---|---|---|---|---|---|---|---|---|---|---|---|---|
| | Activity atone (ug/ml) | | | | Combination with delmopinol (FIC) | | | | Evaluation in Decapinol | | | |
| Compound | Standard | | Decapinol* base | | Standard | | Decapinol* base | | 1:1 Ratio | | Adjusted ratio^ | |
| | MIC | MBC | MIC | MBC | FICi | FBCI | FICi | FBCi | FICi | FBCi | FICi | FBCi |
| CPC | 0.98 | 0.98 | 0.49 | 0.98 | 0.63p | 0.68p | 0.99 | 0.65p | 1 | 2 | 1.25 | 1.25 |
| | | | | | | | | | | | | |
| Delmopinol | 500 | 500 | 120 | 120 | | | | | | | | |

* No delmopinol or sodium benzoate present
^ Ratios: 50:1 (Del:CPC)
p = potentiation
s = synergy

**Table 12**

| *C. albicans* | | | | | | | | | | | | |
|---|---|---|---|---|---|---|---|---|---|---|---|---|
| | Activity alone (uM) | | | | Combination with delmopinol (FIC) | | | | Evaluation in Decapinol | | | |
| Compound | Standard | | Decapinol* base | | standard | | Decapinol* base | | 1:1 Ratio | | Adjusted ratio^ | |
| | MIC | MBC | MIC | MBC | FICi | FBCi | FICI | FBCi | FICi | FBCi | FICi | FBCi |
| CPC | 2.9 | 2.9 | 1.4 | 2.9 | 0.63p | 0.66p | 0.99 | 0.65p | 1 | 2 | 1.25 | 1.25 |
| | | | | | | | | | | | | |
| Delmopinol | 1840 | 1840 | 460 | 460 | | | | | | | | |

* No delmopinol or sodium benzoate present
^ Ratios: 50:1 (Del:CPC)
p = potentiation
s = synergy

**Table 13**

| Summary of all synergies detected | | | | | | |
|---|---|---|---|---|---|---|
| Compound + delmopinol | *P.gingivalis* | *Prv. Nigrescens* | *S. aureus* | *E. faecalis* | *E. coli* | *C. albicans* |
| CPC | potentiation | synergy | synergy | potentiation | potentiation | potentiation |

As displayed in Table 13, the data from the above tables show that compositions comprising delmopinol and CPC in combination, compared to just delmopinol alone or CPC alone, surprisingly have potentiating or synergistic antimicrobial effects on a range of microbes. The reformulated decapinol test shows that the observed potentiation or synergistic antimicrobial activity is not due to the excipients that are present in Decapinol solution.

Accordingly, compositions of the present invention comprising the combination of a morpoholino compound of Formula (I) and a cetylpyridinium salt display an unexpected advantage at treating or preventing infection caused by a range of microbes associated with the pathological colonisation of wounds and periodontal lesions.

## Claims

1. A composition comprising a morpholino compound in combination with a cetylpyridinium salt, the morpholino compound having the general formula (I)

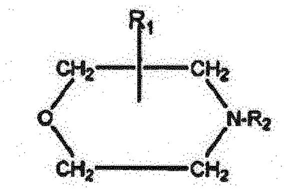

(I)

wherein R1 is a straight or branched alkyl group containing 8 to 16 carbon atoms at the 2- or 3- position of the morpholino ring, and R2 is a straight or branched alkyl group containing 2 to 10 carbon atoms, substituted with a hydroxyl group except in the alpha-position, or pharmaceutically acceptable salts thereof, for use in the treatment or prevention of an infection in a patient caused by *Staphylococcus spp,* wherein the infection is in a wound of the skin or mucosa of a patient.

2. The composition for use according to claim 1 , wherein the sum of the carbon atoms in the groups R1 and R2 is at least 10, preferably between 10 and 20.

3. The composition for use according to claim 1 or claim 2, wherein R2 terminates with the hydroxyl group.

4. The composition for use according to any preceding claim, wherein the morpholino compound is 3-(4-propyl-heptyl)-4-(2-hydroxyethyl) morpholine.

5. The composition for use of any preceding claim, wherein the infection is caused by *S. aureus.*

6. The composition for use according to any preceding claim, wherein the cetylpridinium salt is a halide salt.

7. The composition for use according to any preceding claim, wherein the cetylpyridinium salt is cetylpyridinium chloride.

8. The composition for use of any of the preceding claims, wherein said composition is in the form of a gel, foam, cream, emollient, spray, wipe mouthwash, toothpaste, chew, or gum.

9. A composition for use as defined in any preceding claim, for use in treating or preventing a post-operative infection in or on the skin or mucosa of a patient.

10. A composition for use as defined in any of claims 1 to 8, for use pre-operatively to reduce the amount of infection-causing microbes present in or on the skin or the mucosa of the patient.

**Patentansprüche**

1. Eine Zusammensetzung, umfassend eine Morpholinverbindung in Kombination mit einem Cetylpyridinsalz, wobei die Morpholinverbindung die allgemeine Formel (I) aufweist

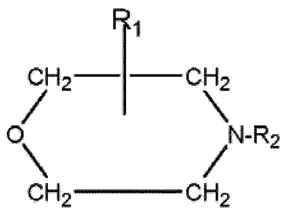

(I)

wobei R1 eine gerade oder verzweigte Alkylgruppe ist, die 8 bis 16 Kohlenstoffatome an der 2- oder 3-Position des Morpholinrings enthält, und R2 eine gerade oder verzweigte Alkylgruppe ist, die 2 bis 10 Kohlenstoffatomen enthält, substituiert mit einer Hydroxylgruppe, außer in der Alpha-Position, oder pharmazeutisch verträgliche Salze davon, zur Verwendung bei der Behandlung oder Vorbeugung einer Infektion bei einem Patienten, hervorgerufen durch *Staphylococcus spp,* wobei sich die Infektion in einer Wunde der Haut oder Schleimhaut eines Patienten befindet.

2. Die Zusammensetzung zur Verwendung nach Anspruch 1, wobei die Summe der Kohlenstoffatome in den Gruppen R1 und R2 mindestens 10, vorzugsweise zwischen 10 und 20 beträgt.

3. Die Zusammensetzung zur Verwendung nach Anspruch 1 oder Anspruch 2, wobei R2 mit der Hydroxylgruppe endet.

4. Die Zusammensetzung zur Verwendung nach einem der vorhergehenden Ansprüche, wobei es sich bei der Morpholinverbindung um 3-(4-Propylheptyl)-4-(2-hydroxyethyl)morpholin handelt.

5. Die Zusammensetzung zur Verwendung nach einem der vorhergehenden Ansprüche, wobei die Infektion durch *S. aureus* hervorgerufen wird.

6. Die Zusammensetzung zur Verwendung nach einem der vorhergehenden Ansprüche, wobei es sich bei dem Cetylpyridinsalz um ein Halogenidsalz handelt.

7. Die Zusammensetzung zur Verwendung nach einem der vorhergehenden Ansprüche, wobei es sich bei dem Cetylpyridinsalz um Cetylpyridinchlorid handelt.

8. Die Zusammensetzung zur Verwendung nach einem der vorhergehenden Ansprüche, wobei die Zusammensetzung in Form eines Gels, Schaumes, einer Creme, eines Emolliens, Sprays, Tuchs, einer Mundspülung, Zahnpasta, eines Kaumittels oder Gummis vorliegt.

9. Eine Zusammensetzung zur Verwendung wie in einem der vorhergehenden Ansprüche definiert, zur Verwendung bei der Behandlung oder Vorbeugung einer post-operativen Infektion in oder auf der Haut oder Schleimhaut eines Patienten.

10. Eine Zusammensetzung zur Verwendung wie in einem der Ansprüche 1 bis 8 definiert, zur Verwendung vor einer Operation, um die Anzahl der Infektion auslösenden Mikroben, die sich in oder auf der Haut oder Schleimhaut des Patienten befinden, zu reduzieren.

**Revendications**

1. Composition comprenant un composé morpholino en combinaison avec un sel de cétylpyridinium, le composé

morpholino ayant la formule générale (I)

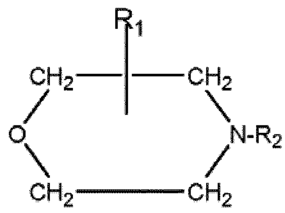

(I)

où R1 est un groupe alkyle linéaire ou ramifié contenant 8 à 16 atomes de carbone à la position 2 ou 3 du cycle morpholino, et R2 est un groupe alkyle linéaire ou ramifié contenant 2 à 10 atomes de carbone, substitué avec un groupe hydroxyle sauf dans la position alpha, ou des sels pharmaceutiquement acceptables de celui-ci, destinée à être utilisée dans le traitement ou la prévention d'une infection chez un patient causée par *Staphylococcus spp,* où l'infection est dans une plaie de la peau ou d'une muqueuse d'un patient.

**2.** Composition destinée à être utilisée selon la revendication 1, où la somme des atomes de carbone dans les groupes R1 et R2 est d'au moins 10, de préférence entre 10 et 20.

**3.** Composition destinée à être utilisée selon la revendication 1 ou la revendication 2, où R2 se termine avec le groupe hydroxyle.

**4.** Composition destinée à être utilisée selon l'une quelconque des revendications précédentes, où le composé morpholino est la 3-(4-propyl-heptyl)-4-(2- hydroxyéthyl)morpholine.

**5.** Composition destinée à être utilisée selon l'une quelconque des revendications précédentes, où l'infection est causée par *S. aureus.*

**6.** Composition destinée à être utilisée selon l'une quelconque des revendications précédentes, où le sel de cétylpyridinium est un sel halogénure.

**7.** Composition destinée à être utilisée selon l'une quelconque des revendications précédentes, où le sel de cétylpyridinium est le chlorure de cétylpyridinium.

**8.** Composition destinée à être utilisée selon l'une quelconque des revendications précédentes, où ladite composition est sous forme d'un gel, d'une mousse, d'une crème, d'un émollient, d'un spray, d'un bain de bouche à essuyer, d'une pâte dentifrice, d'un produit à mâcher ou d'une gomme.

**9.** Composition destinée à être utilisée telle que définie dans l'une quelconque des revendications précédentes, destinée à être utilisée dans le traitement ou la prévention d'une infection postopératoire dans ou sur la peau ou une muqueuse d'un patient.

**10.** Composition destinée à être utilisée telle que définie dans l'une quelconque des revendications 1 à 8, destinée à être utilisée de manière préopératoire pour réduire la quantité de microbes causant des infections présente dans ou sur la peau ou la muqueuse du patient.

Figure 1

# Identification of synergy

**MIC data transformed into a**

**fractional inhibitory concentration.**

FICA = MIC of A with B / MIC of A

FIC index = FICA + FICB

**FIC index values:**

≤ 0.5 = **SYNERGY**

≥ 0.51- ≤ 0.75 = **POTENTIATION**

≥ 0.76 – 4.0 = **INDIFFERENT**

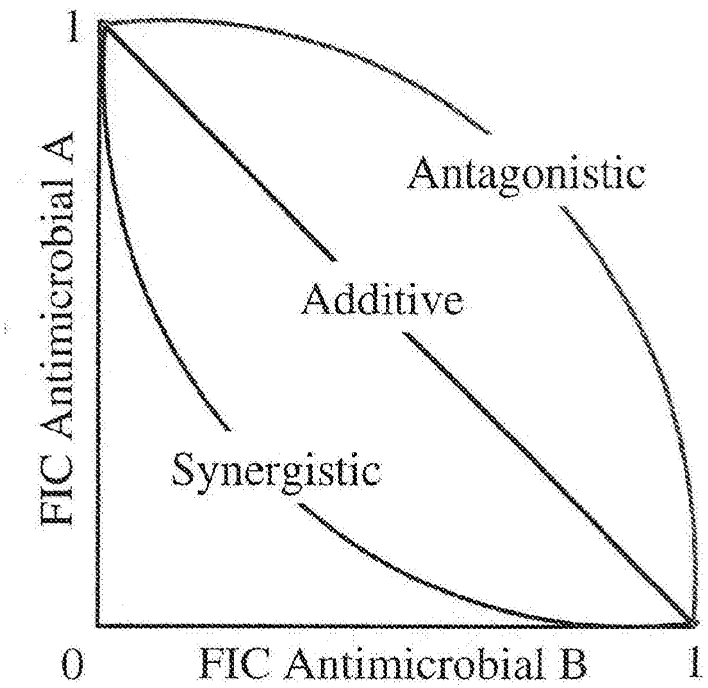

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 06082393 A
- US 5147632 A
- WO 2007060413 A
- WO 2009013475 A
- JP 2002256155 B
- JP 8151325 B
- US 20030211053 A
- WO 9208442 A
- US 4636382 A
- WO 2007057681 A
- WO 2007091009 A
- US 4894221 A
- US 5082653 A


### Non-patent literature cited in the description


- **BERESWILL et al.** *FEMS Immunology and Medical Microbiology,* 1999, vol. 24, 189-92
- **JAMES et al.** *Wound Rep Reg.,* 2008, vol. 16, 37-44
- *CHEMICAL ABSTRACTS,* 79874